(19) Europäisches Patentamt
European Patent Office
Office européen des brevets

(11) **EP 2 957 290 A1**

(12) **DEMANDE DE BREVET EUROPEEN**

(43) Date de publication:
**23.12.2015 Bulletin 2015/52**

(21) Numéro de dépôt: **15172682.5**

(22) Date de dépôt: **18.06.2015**

(51) Int Cl.:
*A61K 36/9066* (2006.01)     *A61K 35/56* (2015.01)
*A61K 9/00* (2006.01)     *A61P 19/02* (2006.01)
*A61P 29/00* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **19.06.2014 BE 201400467**

(71) Demandeur: **Pharco**
**1180 Uccle (BE)**

(72) Inventeurs:
• WYVEKENS, Guy
  1380 LASNE (BE)
• MOTTE, François
  1380 LASNE (BE)
• GODERIS, Karel
  1709 DILBEEK (BE)

(74) Mandataire: **Gevers Patents**
**Intellectual Property House**
**Holidaystraat 5**
**1831 Diegem (BE)**

(54) **COMPOSITION À BASE D'HUILE DE KRILL ET D'EXTRAIT DE CURCUMA**

(57) Composition pharmaceutique ou parapharmaceutique orale à base d'huile de krill et de curcuminoïde comprenant :
- 39,0% en poids d'huile de krill ;
- 26,0 % en poids d'un extrait de curcuma;

et un mélange d'agents de dispersion comprenant :
- 19,5% en poids de triglycéride;
- 6,5% en poids de glycol;
- 4,0% en poids de polyglycérol;
- 2,6% en poids de lécithine; et
- 1,3% en poids de phosphatidylcholine,

les teneurs ci-dessus étant exprimées par rapport au poids total de la composition.

EP 2 957 290 A1

**Description**

La présente invention porte sur une composition pharmaceutique ou parapharmaceutique orale à base d'huile de krill et d'extrait de curcuma destinée aux mammifères, et en particulier à l'être humain.

L'huile de krill est entre autre réputée pour contribuer au maintien de la santé cardiovasculaire, à la réduction des taux de cholestérol LDL et à l'augmentation des taux de cholestérol HDL, à la réduction des triglycérides/triacylglycérols sériques, généralement conjointement avec une thérapie conventionnelle aux statines, à l'atténuation de la douleur associée à l'arthrose et à la réduction des niveaux de protéines C-réactives qui sont des marqueurs cliniques de l'inflammation, ainsi qu'au soulagement des symptômes de la dysménorrhée et du syndrome prémenstruel. L'huile de krill permet en outre de réduire l'accumulation de graisses dans l'organisme et ainsi améliorer la perte de poids.

Le curcuma est la source principale des curcuminoïdes, et en particulier la curcumine. Ces composés sont quant à eux reconnus pour leur action curative et préventive sur le cancer, de par leurs vertus anti-oxydantes, pour leurs effets anti-inflammatoires et anti-rhumatismaux, pour leur action bénéfique sur le cholestérol, c'est-à-dire une réduction des taux de cholestérol LDL et une augmentation des taux de cholestérol HDL. Les curcuminoïdes soutiennent par ailleurs les processus de détoxifications hépatiques. Les curcuminoïdes peuvent en outre soulager les symptômes du côlon irritable et permettent en outre le traitement de la dyspepsie et des ulcères gastro-intestinaux.

L'extrait de curcuma comprenant les curcuminoïdes sont tirés de la plante et généralement mis sous une forme pulvérulente séchée colorante de couleur rouge prononcée et soutenue.

Actuellement, une telle composition existe sous forme d'une composition unique dans laquelle la quantité en curcuma [quantité $R_C$ (en %) par rapport au poids total de la composition] est très inférieure à celle de l'huile de krill [quantité $R_k$ (en %) par rapport au poids total de la composition]. Cette composition présente typiquement dans un rapport $R_c/R_k$ < 1/100, ce qui indique une solubilité faible des curcuminoïdes dans l'huile de krill.

Cette composition est une composition homogène stable dans laquelle les curcuminoïdes de l'extrait de curcuma sont présents sous la forme de phytosomes de phospholipides, lesquels phospholipides sont issus de l'huile de krill.

Toutefois, actuellement, lorsque la prise de ces deux composés en quantités substantiellement égales est recommandée, elle se fait de manière séparée par la prise d'huile de krill et de l'extrait de curcuma.

Par les termes « quantités substantiellement équivalentes », il faut entendre au sens de la présente invention que la quantité en huile de krill est égale à celle de l'extrait de curcuma, de sorte que l'on ait :

$$R_k \text{ (en \%)} = R_c \text{ (en \%)} \pm D,$$
$$\text{et } R_k + R_c < 100\%$$

, avec D (en %) compris dans une plage allant de 0 à 20, de préférence de 0 à 10.

Cette administration séparée est en réalité le résultat du fait que l'huile de krill est un liquide hydrophobe tandis que les curcuminoïdes, qui par définition sont hydrosolubles et hydrophiles, sont disponibles sous une forme pulvérulente ou dissous dans une solution aqueuse : ces derniers présentent donc peu d'affinité avec les graisses. Dès lors, lorsque ces deux composants sont mélangés ensemble dans les rapports $R_k$ et $R_c$ susmentionnés, ils forment une pâte visqueuse hétérogène qui n'est pas stable et qui rend la mise en oeuvre d'une telle composition difficile à cause de la nature très colorante persistante des curcuminoïdes.

Il en résulte que la chaîne de formulation de cette composition est contaminée par ce colorant puissant, ce qui rend cette chaîne inexploitable pour d'autres applications pharmaceutiques similaires.

En conséquence, une formulation d'huile de krill combinée à un extrait de curcuma en quantités substantiellement égales et dans laquelle les curcuminoïdes du curcuma sont présents sous forme de phytosomes n'a encore jamais vu le jour.

De plus, outre le mélange stable qui n'a pas encore pu être obtenu, la difficulté d'obtenir un mélange homogène pour un conditionnement en dosage médicamenteux ou para-médicamenteux n'est en outre pas négligeable.

Par ailleurs, si un tel mélange était obtenu, il faudrait encore qu'il soit stable dans le temps et qu'il ne donne pas naissance à des produits de dégradation.

Dès lors qu'il est reconnu qu'un des facteurs importants liés au succès commercial d'un produit pharmaceutique ou parapharmaceutique, par exemple un médicament ou un complément alimentaire, réside dans le fait que la prise de ce produit soit avant tout simple et peu contraignante, il existe un besoin réel de disposer d'une composition unique à base d'huile de krill et d'extrait de curcuma en quantités substantiellement équivalentes qui soit plus simple à utiliser et dont la prise soit moins lourde pour le consommateur qui la consomme que la composition actuellement disponible sur le marché, et ce, tout en restant exploitable à l'échelle industrielle et donc économiquement rentable. Ce qui signifie en outre que la composition doit être stable dans le temps et ne doit pas se dégrader.

Cet objectif est atteint par la mise au point d'une composition telle que mentionnée au début comprenant :

- de l'huile de krill dans une quantité comprise dans une plage allant de 25,0% à 40,0% en poids par rapport au poids total de la composition ;
- un extrait de curcuma dans une quantité comprise dans une plage allant de 15,0% à 30,0% en poids par rapport au poids total de la composition, ledit extrait de curcuma comprenant au moins un curcuminoïde, ledit au moins un curcuminoïde étant de préférence sous forme d'un complexe avec au moins un phospholipide, et un mélange d'agents de dispersion comprenant :
- au moins un glycéride et/ou au moins un ester de glycéride dans une plage allant de 2,5% à 35,0% en poids par rapport au poids total de la composition ; et
- au moins un glycol dans une quantité comprise dans une plage allant de 3,0% à 10,0% en poids par rapport au poids total de la composition.

Selon la présente invention, il a été possible par le choix judicieux d'agents de dispersion dans des quantités optimisées de produire pour des quantités $R_c$ et $R_k$ substantiellement identiques, une composition comprenant des phytosomes de curcuminoïdes avec les phospholipides de l'huile de krill, ce qui permet enfin de disposer d'une composition unique comprenant les deux substances actives, laquelle est stable dans le temps, qui peut être distribuée de manière homogène en gélule et en capsule ou autre et qui peut être réellement produite dans des chaînes de production industrielles.

L'avantage d'une telle composition est double : ainsi, à côté du fait que la composition homogène selon l'invention soit stable dans le temps et permette une production à l'échelle industrielle sous différentes formes galéniques, le fait que les curcuminoïdes de l'extrait de curcuma soient encapsulés dans une membrane de phospholipides pour former ainsi les phytosomes rend la composition particulièrement avantageuse dans la mesure où ces curcuminoïdes, encapsulés dans les phytosomes, ne sont plus libres, ce qui permet de disposer d'une composition qui ne tâche pas la chaîne de fabrication.

Ainsi, la stabilité de la composition permet son exploitation à l'échelle industrielle, à savoir que la composition selon l'invention présente donc une formulation reproductible et homogène dans chaque dose produite.

La composition selon la présente invention permet donc de combiner réellement l'action de l'huile de krill à celle de l'extrait de curcuma dans des quantités équivalentes sous la forme d'une composition homogène et stable une fois ingérée, et dont les curcuminoïdes présents sous forme de phytosomes dans la composition selon l'invention présentent une biodisponibilité par voie orale améliorée.

En effet, il est connu que les curcuminoïdes sont très peu biodisponibles par voie orale. A titre d'exemple, chez l'homme, lorsque des doses orales de curcuminoïdes sont administrées dans des proportions allant de 4g à 8g, les concentrations plasmatiques mesurées sont comprise dans une plage allant de 0,41g/L à 1,75g/L (voir la Lettre de l'Institut Européen de Physionutrition, n° 14).

La biodisponibilité améliorée des curcuminoïdes par voie orale est rendue possible par la stabilité *in vivo* des phytosomes présents dans la composition selon l'invention.

Dès lors que la composition selon l'invention est à base d'un extrait de curcuma dont les curcuminoïdes présentent une biodisponibilité qui est améliorée par voie orale, cette dernière permet alors une assimilation de curcuminoïdes à des doses réduites, ce qui constitue d'une part une perspective de gain en termes de coût, dès lors qu'une dose moindre doit être absorbée, et, d'autre part, une diminution des effets secondaires liés à la prise de curcuminoïdes, en particulier dans le cadre d'un traitement de longue durée dès lors que les seules plaintes à fortes doses sont des irritations stomacales que l'on pourrait dès lors supprimer ou pour le moins réduire par l'utilisation de la composition selon l'invention dans le cadre d'une consommation prévue sur une longue durée.

Avantageusement, ledit au moins un curcuminoïde est de la curcumine.

De manière préférentielle, ledit au moins un glycol est du propylène glycol.

De préférence, ledit au moins un glycéride est un triglycéride qui présente une chaîne aliphatique comprenant entre 6 et 12 atomes de carbone [C6-C12].

De manière avantageuse, ledit au moins un glycéride est un triglycéride présent dans ladite composition dans une quantité comprise dans une plage allant de 5,0% à 30,0% en poids par rapport au poids total de la composition.

Eventuellement, ledit au moins un ester de glycéride est un polyglycérol présent dans ladite composition dans une quantité comprise dans une plage allant de 2,5% à 10,0%, de préférence entre 2,5% et 5,0%, en poids par rapport au poids total de la composition.

De manière alternative, ledit au moins un polyglycérol est du polyglycérol dioléate.

De manière avantageuse, une fraction d'au moins 50% en poids de phosphatidylcholine provient de la lécithine de soja.

En outre, dans la composition selon l'invention, le krill peut être un krill antarctique *Euphausia Superba*.

De préférence, ledit au moins un curcuminoïde est de la curcumine ou un dérivé de la curcumine.

En outre, la composition selon l'invention peut comprendre de la phosphatidylcholine dans une quantité comprise dans une plage allant de 2,0% à 3,0% en poids par rapport au poids total de la composition.

Avantageusement, la phosphatidylcholine est d'origine animale ou végétale. La phosphatidylcholine peut en outre provenir d'au moins un végétal, par exemple du soja et/ou de la fleur de tournesol.

Dans un mode de réalisation particulier de la composition selon l'invention, cette dernière est encapsulée dans une capsule présentant une enveloppe comprenant de la gélatine animale ou de synthèse et du glycérol dans un rapport pondéral gélatine : gylcérol compris dans une plage allant de 1,0 : 1,0 à 2,0 : 1,0.

L'encapsulation dans une capsule de gélatine présente plusieurs avantages : la gélatine permet une meilleure intégration de la composition par le corps, étant donné que la gélatine est une protéine qui est destinée à être dégradée facilement par le système gastro-intestinal. Ensuite, l'enveloppe gélatineuse est étanche à l'air et permet d'empêcher l'oxydation de la composition. Enfin, l'enveloppe de gélatine, de par ses propriétés hermétiques, permet de conserver les goûts les plus désagréables provenant de la composition (en particulier le goût de l'huile de krill qui s'apparente à celui de l'huile de foie de morue).

D'autres formes de la composition selon l'invention sont indiquées dans les revendications annexées.

La présente invention porte en outre sur la composition selon l'invention, pour une utilisation par voie orale dans le cadre du traitement préventif et/ou curatif des maladies inflammatoires et/ou dégénératives, en particulier de l'arthrose.

Cette utilisation est rendue possible grâce à l'action anti-inflammatoire reconnue de l'huile de krill et du curcuminoïde.

En effet, d'une part, l'huile de krill est réputée pour être riche en phospholipides, par exemple en phospahtidylcholine liées à des acides gras $\omega 3$, par exemple l'acide eicosapentaénoïque (EPA), l'acide docosahexaénoïque (DHA), l'acide alpha-linoléique, ou un mélange de ceux-ci.

Les acides gras $\omega 3$ et les phospholipides sont connus comme stimulants de la production chez les mammifères, et en particulier l'être humain, de prostaglandines E1 qui inhibent la synthèse des prostaglandines E2 aux propriétés pro-inflammatoires.

D'autre part, l'action anti-inflammatoire des curcuminoïdes est comparable à celle de la cortisone, de la phénylbutazone (utilisée dans le traitement de l'arthrite rhumatoïde) et des anti-inflammatoires non-stéroïdiens. La curcumine agit en inhibant des enzymes qui participent à la synthèse des substances inflammatoires - elles-mêmes dérivées de l'acide arachidonique - comme les prostaglandines E2 et les leucotriènes. Les curcuminoïdes participent à la diminution du relâchement des médiateurs chimiques des cellules et des neutrophiles.

Cependant, dans le cadre de la présente invention, il a été observé de manière tout à fait surprenante que le mélange unique composé d'huile de krill et du curcuminoïde lorsqu'ils sont présents dans le rapport de quantités revendiquées, c'est-à-dire lorsque le $R_c$ est substantiellement égal au $R_k$, présente un effet anti-inflammatoire synergique de sorte qu'il ne pouvait être anticipé à partir de propriétés connues de l'huile de krill et du curcuminoïde.

Dès lors que la composition selon l'invention présente une activité anti-inflammatoire améliorée, cette dernière peut être administrée à des doses réduites, ce qui contribue à une perspective de gain en termes de coût de cure ou de traitement dans le cadre de l'application anti-inflammatoire, dès lors qu'une dose moindre doit être absorbée pour obtenir le même effet que celui obtenu lorsque les deux composés sont pris séparément.

Cette activité anti-inflammatoire améliorée est assurée par la prise simultanée de l'huile de krill et du curcuminoïde aux quantités revendiquées et qui est donc rendue possible par la composition selon l'invention.

De préférence, la composition selon l'invention comprend l'huile de krill dans une quantité comprise dans une plage allant de 30,0% à 40,0%, avantageusement de 35,0% à 40,0%, de manière préférentielle de 36,0% à 40,0%, de manière avantageuse de 37,0% à 40,0%, de manière plus préférentielle de 38,0% à 40,0%, de manière encore plus préférentielle, de 39,0% à 40,0% en poids par rapport au poids total de la composition.

De préférence, la composition selon l'invention comprend l'extrait de curcuma dans une quantité comprise dans une plage allant de 15,0% à 30,0%, avantageusement de 20,0% à 30,0%, de manière préférentielle de 21,0% à 30,0%, de manière avantageuse de 22,0% à 30,0%, de manière plus préférentielle de 23,0% à 30,0%, de manière encore plus préférentielle de 24,0% à 30,0%, de manière encore plus préférentielle de 25,0% à 30,0% en poids par rapport au poids total de la composition.

De manière alternative, la composition selon l'invention comprend l'extrait de curcuma dans une quantité comprise dans une plage allant de 26,0% à 30,0%, de préférence de 26,0% à 29,0%, de manière avantageuse, de 26,0% à 28,0%, de manière plus préférentielle de 26,0% à 27,0% en poids par rapport au poids total de la composition.

Optionnellement, l'extrait de curcuma comprenant au moins un curcuminoïde dans une quantité comprise dans une plage allant de 20,0% à 95,0%, de manière avantageuse de 20,0 à 85%, de préférence 20,0% à 75,0%, avantageusement de 20,0% à 50,0%, éventuellement de 20,0% à 40,0% en poids par rapport au poids total dudit extrait de curcuma.

De préférence l'extrait de curcuma comprend de la curcumine dans une quantité comprise dans une plage allant de 20,0% à 95,0% en poids par rapport au poids total dudit extrait de curcuma.

La présente invention se rapporte aussi à une composition alimentaire, un complément alimentaire, ou un aliment à base de la composition selon l'invention.

Par les termes « complément alimentaire », il faut entendre au sens de la présente invention une denrée alimentaire dont le but est de fournir un complément de nutriments ayant un effet nutritionnel et/ou physiologique.

D'autres formes de réalisation de la composition alimentaire, du complément alimentaire, ou de l'aliment suivant l'invention sont indiquées dans les revendications annexées.

La présente invention porte aussi sur une composition pharmaceutique à base de la composition selon l'invention.

D'autres formes de réalisation de la composition pharmaceutique suivant l'invention sont indiquées dans les revendications annexées.

La présente invention concerne par ailleurs une utilisation de la composition selon l'invention comme complément alimentaire.

D'autres formes d'utilisation de la composition alimentaire comme complément alimentaire suivant l'invention sont indiquées dans les revendications annexées.

La présente invention va maintenant être décrite plus en détail au moyen de l'exemple de réalisation ci-dessous donné à titre non-limitatif.

**Exemple**

Une composition orale de poids total de 770,0mg a été formulée à l'échelle industrielle et encapsulée dans une enveloppe de gélatine de 301,6mg.

Par capsule, la composition présente la formulation suivante :

| Composition | | |
|---|---|---|
| Composants | Poids (en mg) | % (Par rapport au poids total de la composition) |
| Krill d'antarctique | 300,0 | 39,0% |
| Extrait de curcuma[1] | 200,0 | 26,0% |
| Triglycéride à chaîne moyenne[2] | 150,0 | 19,5% |
| Propylène glycol monolaurate | 50,0 | 6,5% |
| Polyglycérol dioléate | 30,0 | 4% |
| Lécithine de tournesol | 20,0 | 2,6% |
| Phosphatidylcholine[3] | 20,0 | 2,6% |
| Poids total de la composition: 770,0mg | | |
| Capsule | | |
| Composants | Poids (en mg) | |
| Gélatine de poisson | 188,0 | |
| Glycérol | 112,8 | |
| Oxyde de fer[4] | 0,8 | |
| Poids total de la capsule: 301,6mg + 770,0mg = 1071,6mg | | |
| [1] L'extrait de curcuma est constitué d'un complexe C :P qui est un phytosome formé entre au moins une molécule de curcumine (C) et au moins une molécule de phospholipide (P) qui sont liées chimiquement entre elles. Dans le cadre de la présente invention, ladite au moins une molécule de curcuminoïdes est encapsulée dans une capsule formée par au moins une couche de phospholipides, lesquels phospholipides provenant de l'huile de krill ou d'une autre source. Le complexe C :P comprend de la curcumine dans une teneur comprise entre 20,0% et 95,0% en poids par rapport au poids total du complexe C : P ; [2] [C6-C12] ; [3] 50% en poids de la lécithine est de la lécithine de soja ; [4] Colorant rouge | | |

Dans cet exemple, l'extrait de curcuma est un complexe C :P comprend de la curcumine dans une teneur comprise entre 20,0% et 95,0% en poids par rapport au poids total du complexe C : P.

Par capsule, les apports énergétiques ainsi que les apports en graisses, en sucres, et en curcumine (C) sont définis ci-dessous :

| Apports | |
|---|---|
| Kcal | 5,9 |
| KJ | 24,5 |
| Protéines | 177,0mg |
| Hydrates de carbone | 113,0mg |
| Graisses | 528,0mg |
| Phospholipides | 135,0mg |
| acides gras $\omega 3$ | 72,0mg |
| EPA | 36,0mg |
| DHA | 20,0mg |
| Complexe C :P[1] | 200,0mg |

Il est bien entendu que la présente invention n'est en aucune façon limitée aux formes de réalisations décrites ci-dessus et que bien des modifications peuvent y être apportées sans sortir du cadre des revendications annexées.

Par exemple dans le cadre de l'utilisation de la composition selon la présente invention, la dose prescrite de prise de la composition selon l'invention est de 1 à 4 capsules (de 1071,6mg comprenant 770 mg de la composition selon l'invention) par jour.

**Revendications**

1.  Composition pharmaceutique ou parapharmaceutique orale à base d'huile de krill et d'extrait de curcuma destinée aux mammifères, et en particulier à l'être humain, l'huile de krill et le curcuma étant présents en quantités substantiellement égales, ladite composition comprenant :

    - de l'huile de krill dans une quantité comprise dans une plage allant de 25,0% à 40,0% en poids par rapport au poids total de la composition ;
    - un extrait de curcuma dans une quantité comprise dans une plage allant de 15,0% à 30,0% en poids par rapport au poids total de la composition, ledit extrait de curcuma comprenant au moins un curcuminoïde, et un mélange d'agents de dispersion comprenant :
    - au moins un glycéride et/ou au moins un ester de glycéride dans une plage allant de 2,5% à 35,0% en poids par rapport au poids total de la composition ; et
    - au moins un glycol dans une quantité comprise dans une plage allant de 3,0% à 10,0% en poids par rapport au poids total de la composition.

2.  Composition selon la revendication 1, dans laquelle ledit extrait de curcuma comprenant au moins un curcuminoïde dans une quantité comprise dans une plage allant de 20,0% à 95,0% en poids par rapport au poids total dudit extrait de curcuma.

3.  Composition selon la revendication 1 ou 2, dans laquelle ledit au moins un curcuminoïde est de la curcumine ou un dérivé de la curcumine.

4.  Composition selon l'une quelconque des revendications précédentes, dans laquelle ledit au moins un curcuminoïde est présent sous forme d'un complexe avec au moins un phospholipide.

5.  Composition selon l'une quelconque des revendications précédentes, comprenant de la phosphatidylcholine dans une quantité comprise dans une plage allant de 2,0% à 3,0% en poids par rapport au poids total de la composition.

**6.** Composition selon la revendication 5, dans laquelle ladite phosphatidylcholine est d'origine animale ou végétale.

**7.** Composition selon la revendication 6, dans laquelle ladite phosphatidylcholine provient d'au moins un végétal qui est du soja et/ou de la fleur de tournesol.

**8.** Composition selon l'une quelconque des revendications précédentes, **caractérisée en ce que** ledit au moins un glycéride est un triglycéride présent dans ladite composition dans une quantité comprise dans une plage allant de 5,0% à 30,0% en poids par rapport au poids total de la composition.

**9.** Composition selon l'une quelconque des revendications précédentes, **caractérisée en ce que** ledit au moins un ester de glycéride est un polyglycérol présent dans ladite composition dans une quantité comprise dans une plage allant de 2,5% à 10,0% en poids par rapport au poids total de la composition.

**10.** Composition selon l'une quelconque des revendications précédentes, **caractérisée en ce qu'**elle est encapsulée dans une capsule présentant une enveloppe comprenant de la gélatine animale ou de synthèse et du glycérol dans un rapport pondéral gélatine : gylcérol compris dans une plage allant de 1,0 : 1,0 à 2,0 : 1,0.

**11.** Composition selon l'une quelconque des revendications 1 à 10, pour une utilisation pour le traitement par voie orale, préventif et/ou curatif, des maladies inflammatoires et/ou dégénératives, en particulier de l'arthrose.

**12.** Utilisation de ladite composition selon l'une quelconque des revendications 1 à 10, comme complément alimentaire.

**13.** Composition alimentaire, complément alimentaire, ou aliment à base de la composition selon l'une quelconque des revendications 1 à 10.

**14.** Composition pharmaceutique à base de la composition selon l'une quelconque des revendications 1 à 10.

Europäisches
Patentamt

European
Patent Office

Office européen
des brevets

## RAPPORT DE RECHERCHE EUROPEENNE

Numéro de la demande

EP 15 17 2682

| | DOCUMENTS CONSIDERES COMME PERTINENTS | | |
|---|---|---|---|
| Catégorie | Citation du document avec indication, en cas de besoin, des parties pertinentes | Revendication concernée | CLASSEMENT DE LA DEMANDE (IPC) |
| X | WO 2012/120378 A1 (OLYMPIC SEAFOOD AS [NO]; BRUHEIM INGE [NO]; GRIINARI MIKKO [FI]; REMOY) 13 septembre 2012 (2012-09-13) * revendications; exemples * ----- | 1-14 | INV. A61K36/9066 A61K35/56 A61K9/00 A61P19/02 A61P29/00 |
| X | US 2011/104297 A1 (BRUHEIM INGE [NO] ET AL) 5 mai 2011 (2011-05-05) * alinéas [0113] - [0115]; revendications * ----- | 1-14 | |
| X | WO 2011/011604 A2 (U S NUTRACEUTICALS LLC D B A VALENSA INTERNAT [US]) 27 janvier 2011 (2011-01-27) * alinéa [0049]; revendications * ----- | 1-14 | |
| | | | DOMAINES TECHNIQUES RECHERCHES (IPC) A61K A61P |

Le présent rapport a été établi pour toutes les revendications

| Lieu de la recherche | Date d'achèvement de la recherche | Examinateur |
|---|---|---|
| Munich | 15 octobre 2015 | Friederich, Martin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.....................................................................
& : membre de la même famille, document correspondant

EPO FORM 1503 03.82 (P04C02)

**EP 2 957 290 A1**

**ANNEXE AU RAPPORT DE RECHERCHE EUROPEENNE
RELATIF A LA DEMANDE DE BREVET EUROPEEN NO.**

EP 15 17 2682

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-10-2015

| Document brevet cité au rapport de recherche | Date de publication | Membre(s) de la famille de brevet(s) | Date de publication |
|---|---|---|---|
| WO 2012120378 A1 | 13-09-2012 | AU 2012226531 A1<br>CA 2829453 A1<br>EP 2683253 A1<br>US 2012231087 A1<br>WO 2012120378 A1 | 10-10-2013<br>13-09-2012<br>15-01-2014<br>13-09-2012<br>13-09-2012 |
| US 2011104297 A1 | 05-05-2011 | US 2011104297 A1<br>US 2014363517 A1 | 05-05-2011<br>11-12-2014 |
| WO 2011011604 A2 | 27-01-2011 | DE 202010017863 U1<br>DE 202010018054 U1<br>EP 2456449 A2<br>EP 2902033 A1<br>KR 20120038526 A<br>KR 20140096168 A<br>US 2011020316 A1<br>US 2013004582 A1<br>US 2013005828 A1<br>US 2013280341 A1<br>US 2013287756 A1<br>US 2014011888 A1<br>WO 2011011604 A2 | 23-11-2012<br>18-10-2013<br>30-05-2012<br>05-08-2015<br>23-04-2012<br>04-08-2014<br>27-01-2011<br>03-01-2013<br>03-01-2013<br>24-10-2013<br>31-10-2013<br>09-01-2014<br>27-01-2011 |

EPO FORM P0460

Pour tout renseignement concernant cette annexe : voir Journal Officiel de l'Office européen des brevets, No.12/82